(19) **Europäisches Patentamt**
**European Patent Office**
**Office européen des brevets**

(11) **EP 4 428 223 A1**

(12) **EUROPEAN PATENT APPLICATION**
published in accordance with Art. 153(4) EPC

(43) Date of publication:
**11.09.2024 Bulletin 2024/37**

(51) International Patent Classification (IPC):
*C12N 1/16* (2006.01)    *A23L 33/14* (2016.01)
*A23L 33/145* (2016.01)    *A23K 10/16* (2016.01)
*A23K 10/18* (2016.01)    *A23L 27/10* (2016.01)
*C12R 1/645* (2006.01)

(21) Application number: 22900482.5

(22) Date of filing: 29.11.2022

(86) International application number:
**PCT/CN2022/135105**

(87) International publication number:
**WO 2023/098678 (08.06.2023 Gazette 2023/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 02.12.2021  CN 202111457132

(71) Applicant: **Angel Yeast Co., Ltd
Yichang, Hubei 443003 (CN)**

(72) Inventors:
• **QIN, Xianwu
Yichang, Hubei 443003 (CN)**

• **YAO, Su
Yichang, Hubei 443003 (CN)**
• **SUN, Yafang
Yichang, Hubei 443003 (CN)**
• **GUO, Tianfen
Yichang, Hubei 443003 (CN)**
• **BAI, Feirong
Yichang, Hubei 443003 (CN)**
• **WANG, Jingwen
Yichang, Hubei 443003 (CN)**
• **CHEN, Liting
Yichang, Hubei 443003 (CN)**

(74) Representative: **karo IP
karo IP Patentanwälte
Kahlhöfer Rößler Kreuels PartG mbB
Postfach 32 01 02
40416 Düsseldorf (DE)**

(54) **HIGH-PROTEIN SACCHAROMYCES CEREVISIAE AND APPLICATION THEREOF**

(57)    Provided is high-protein *Saccharomyces cerevisiae* and an application thereof. The provided *Saccharomyces cerevisiae* AMCC strain is preserved in the China Center for Type Culture Collection (CCTCC) on July 27, 2021, and has a preservation number of CCTCC NO: M 2021941. The provided *Saccharomyces cerevisiae* strain has the characteristics of high biomass, high protein and high RNA.

## Description

## Technical Field

The present invention belongs to the technical field of microorganisms, and more particularly, relates to high-protein *Saccharomyces cerevisiae* and an application thereof.

## Background Art

*Saccharomyces cerevisiae* is a single-celled organism, which is safe and non-pathogenic, with the advantages of short growth cycle, vigorous metabolism, strong fermentation capability and rich nutrients such as a variety of proteins, amino acids, vitamins and life active substances, functions as a very important industrial microorganism in the traditional and modern biotechnical fields, and is widely applied in the production of medicines, fine chemicals, bioenergy, industrial enzymic preparations, feed additives, fermented food, etc.

There are certain differences in nutrients contained in different yeasts. The yeast strains can produce rich protein resources after hydrolysis, which may be used as effective feed proteins for livestock and poultry, aquatic products, ruminant animals and other raised animals, so as to promote healthy breeding of livestock and poultry, improve the productivity of livestock and poultry, improve the fecundity of livestock and poultry, stabilize the quality of feeds, improve the vitality and quality of livestock and poultry products, etc.; or may be used as nutritional multi-functional umami agents and flavor enhancers to endow products with different sensory properties; or may also be used as the best source of high-quality proteins for the human body to improve the human immunity, reduce the fatigue, and provide balanced nutritional supplements for the body. Therefore, high-protein yeast strains are a key factor to obtain high-quality protein sources, which can lay the foundation for the industrial production of yeast extracts, and also meet rigid demands for comprehensive, balanced and sustainable development in feed, food and health care industries.

The prior art CN106399135 provides high-yield protein *Saccharomyces cerevisiae* C20140911, as well as its breeding and culture methods and an application thereof, which is used to prepare better microecological preparations and feed additives, in order to enhance its clinical use effect and serve the food safety and animal husbandry production.

The prior art CN106399135 provides a high-yield protein yeast strain, which is bred by using an adaptive evolution technology. The specific implementation method includes: performing passage culture using a malt extract medium, performing growth tolerance domestication on the yeast strain starting from peptone having a low dose of 1%, increasing a dose of peptone gradually to 8%, and screening the high-yield protein yeast strain after multiple re-screening and passage tests; performing protein content, species and bioinformatics analysis on fermentation broth of this yeast strain, including GO analysis, KEGG metabolic pathway analysis, common protein analysis, clustering analysis of common histone WEGO functional annotation, etc.; selecting single factors such as initial pH, culture temperature, and liquid loading volume for response surface analysis, and determining optimal culture conditions by colony number indexes and a variance analysis simulation equation; and then, performing intestinal microflora analysis of piglets to detect the changes in the number of microorganisms in the ileum, cecum and colon, including Escherichia coli, Salmonella, aerobic bacteria, bifidobacteria, lactobacilli and anaerobic bacteria, and performing production performance analysis according to daily gain, daily feed intake, body weight and other indexes of the piglets.

## Summary of the Invention

A *Saccharomyces cerevisiae* strain in the prior art cannot achieve high biomass, high protein and high RNA performances at the same time. In view of the problems existing in the prior art, the present invention provides a *Saccharomyces cerevisiae* strain with high biomass, high protein and high RNA. Generally, a yeast has an intracellular protein content of more than 40%. At present, a protein content of yeast strains in the reported literatures or patents is up to 62.4%. A yeast strain with a protein content of 68.13% is obtained by natural screening in the present invention, which is higher than a research level of existing literatures or patents.

In a first aspect, the present invention provides a high-protein *Saccharomyces cerevisiae* strain, which is a *Saccharomyces cerevisiae* AMCC 30743 strain (Saccharcmyces cerevisiae AMCC 30743), the *Saccharomyces cerevisiae* AMCC 30743 (Saccharcmyces cerevisiae AMCC 30743) strain being preserved in the China Center for Type Culture Collection (CCTCC) on July 27, 2021, and having a preservation number of CCTCC NO: M 2021941.

Preferably, a 26S rDNA gene sequence of the *Saccharomyces cerevisiae* AMCC 30743 strain is shown in SEQ ID NO.1.

In a second aspect, the present invention provides a fermentation preparation method for the *Saccharomyces cerevisiae* strain, wherein the method includes the following step: culturing the *Saccharomyces cerevisiae* AMCC 30743 strain.

Preferably, the preparation method further includes the following steps:

(1) performing amplified culture on the *Saccharomyces cerevisiae* AMCC 30743 strain; and

(2) adding the product obtained in step (1) to a liquid medium and performing fermented culture at 26-32°C.

In a third aspect, the present invention provides a microbial agent, the microbial agent being obtained from the *Saccharomyces cerevisiae* AMCC 30743 strain.

In a fourth aspect, the present invention provides an application of the *Saccharomyces cerevisiae* AMCC 30743 strain in a yeast extract.

In a fifth aspect, the present invention provides a yeast extract, the yeast extract being prepared from the *Saccharomyces cerevisiae* AMCC 30743 strain or the microbial agent.

In a sixth aspect, the present invention provides a microbial agent, the microbial agent being obtained by fermentation according to the aforesaid preparation method.

Preferably, the microbial agent obtained by culturing in a shake flask for 15-20 h has a biomass of 48-52 g/L; or

preferably, the microbial agent has an intracellular protein content of greater than 65wt% of a dry weight of yeast cells; or

preferably, the microbial agent has an RNA content of greater than 15wt% of the dry weight of the yeast cells; or

preferably, the microbial agent has an intracellular glutamic acid content of greater than 9%; or

preferably, an intracellular succinic acid content of the microbial agent is a measured succinic acid content in a supernatant which were obtained after deionized water is added according to a mass volume ratio of the yeast milk to the deionized water of 1:10 and cells are broken, and the succinic acid content in a supernatant is greater than 460 μg/mL.

In a seventh aspect, the present invention provides an application of the *Saccharomyces cerevisiae* AMCC 30743 strain or the microbial agent or the yeast extract in feeds, food and health care products.

The present invention further provides an application of the *Saccharomyces cerevisiae* AMCC 30743 strain or the microbial agent or the yeast extract in condiments.

The *Saccharomyces cerevisiae* strain provided by the present invention has the characteristics of high biomass, high protein and high RNA, and may be applied to feeds, food, health care and other industries to realize the sustainable development of the industrialization of high-protein hydrolysate.

## Strain preservation information

The *Saccharomyces cerevisiae* AMCC 30743 strain (Saccharomyces cerevisiae AMCC 30743) provided by the present invention is preserved in the China Center for Type Culture Collection (CCTCC) on July 27, 2021, and has a preservation number of CCTCC NO: M 2021941 (a preservation address: Wuhan University, Wuhan, China, postal code: 430072, telephone: 027-68754052).

## Brief Description of the Drawings

FIG. 1 shows determination results of biomasses of yeast strains in Example 3;
FIG. 2 shows determination results of protein contents of the yeast strains in Example 3;
FIG. 3 shows determination results of RNA contents of the yeast strains in Example 3;
FIG. 4 shows determination results of amino acid component contents of yeast strains in Example 4; and
FIG. 5 shows determination results of succinic acid contents in Example 5.

## Detailed Description of the Invention

A *Saccharomyces cerevisiae* strain provided by the present invention has the characteristics of high biomass, high protein and high RNA. The present invention provides a *Saccharomyces cerevisiae* strain. Firstly, six yeast strains each having a specific growth rate of greater than or equal to 0.3 $h^{-1}$ were obtained by isolation, purification and identification from fermented yogurt samples in the Ali area of Xizang; and then, yeast milk was collected by shake flask fermentation, and its physiological and biochemical indexes (including biomass, protein, and RNA) were evaluated. The present invention takes *Saccharomyces cerevisiae* FX-2 as a control strain, and screening criteria were that a biomass reached 110-120% of a biomass of the control strain, a protein content reached 100-110% of a protein content of the control strain, and an RNA content reached 110-120% of an RNA content of the control strain. Finally, glutamic acid content and succinic acid content analysis were performed on a preferred strain, and a screening criterion for the glutamic acid content analysis was that the glutamic acid content at least reached 125-130% of a glutamic acid content of the control strain, and a screening criterion for the succinic acid content analysis was that the succinic acid content reached 110-120% of a succinic acid content of the control strain.

The control *Saccharomyces cerevisiae* FX-2 used in the present invention was preserved in the China Center for Type Culture Collection (CCTCC) (a preservation address: Wuhan University, Wuhan, China) on August 1, 2016, had a preservation number of CCTCC NO: M 2016418, and had been recorded in CN108220175A.

High-protein yeast strains are of a key factor to obtain high-quality protein sources, which can lay the foundation for the industrial production of yeast extracts, and also meet rigid demands for comprehensive, balanced and sustainable development in feed, food and health care industries. The components of a medium involved in the following examples were as follows: YPD medium: 10 g of yeast extract powder, 20 g of glucose, 20 g of peptone, 20 g of agar, and 1000 mL of water, all of which were sterilized at 115°C for 20 min.

The yeast milk in the present invention referred to yeast cells which were obtained by centrifugal removal of a supernatant from the fermentation broth obtained by fermentation, and then washing, suction filtration, pressing filtration, as well as separation and collection.

Table 1 and Table 2 below show source information of reagents and instruments used in the examples of the present invention.

Table 1 Reagent information table

| Reagent | Manufacturer |
| --- | --- |
| Yeast extract powder | Angel yeast |
| Glucose | SCR |
| Peptone | Angel yeast |
| Agar | Guixing Chemical |
| Concentrated sulfuric acid | Xilong Chemical |
| Boric acid | Macklin |
| Methyl red | Macklin |
| Bromocresol green | Macklin |
| Sodium hydroxide | SCR |
| Perchloric acid | Xiongda Chemical |
| Sulfosalicylic acid | JINKO Chemical |
| Sodium citrate | Huahang Chemical |
| 2×PCR Mix | TIANGEN BIOTECH |

Table 2 Instrument information table

| Instrument | Model | Manufacturer |
| --- | --- | --- |
| Analytical balance | ME4002E | METTLER TOLEDO |
| pH meter | PB-10 | Sartorius |
| Rapid moisture meter | MJ33 | METTLER TOLEDO |
| Superclean bench | SKJH-1109 | Shanghai Sukun |
| Constant temperature shaker | ZWYR-2102C | Shanghai Zhicheng |
| Biochemical incubator | SPX-158L | Ningbo Xinzhi |
| Thermostat water bath | HH-2 | Jiangsu Guohua |
| PCR instrument | C1000 | BIO-RAD |
| Gel imaging system | GelDoc™ XR+ | BIO-RAD |
| Centrifuge | DL-5200B-II | Flying Pigeon (Shanghai) |
| Electrophoresis apparatus | EPS-300 | Shanghai Tanon |
| Ultra-violet and visible spectrophotometer | UV2310II | Hangzhou Allsheng |
| Optical microscope | CX43 | OLYMPUS |
| Fully automatic growth curve analyzer | BioscreenC | OY Growth Curves |
| Amino acid analyzer | HT1010 | Qingdao HiTech Innovative |
| Liquid chromatograph | 1260II | Aigilent |

(continued)

| Instrument | Model | Manufacturer |
|---|---|---|
| Electrothermal blowing dry box | DHG-9070A | Shanghai Jinghong |

**Example 1: isolation, purification and identification of yeast strains**

Each fermented milk product sample collected in Shannan, Xizang was dissolved in sterile water and mixed well; a microbial suspension was pipetted and diluted in 10-fold series to prepare a $10^{-5}$ microbial suspension and $10^{-6}$ microbial suspension, which were then coated in a YPD medium and cultured at 30°C for 24-48 h; a yeast morphology in a prepared slide was observed under a microscope, and the characteristics of single colonies on a flat plate were observed at the same time; and a strain with typical yeast colony characteristics was isolated, streaked and purified twice, inoculated in a YPD inclined medium, and stored at 4°C. A total of thirty yeast strains were obtained.

The resulting yeast strain colonies were cheese-like in character, milky white in color, wrinkled on surfaces, wavy on edges, oval in micromorphology, and budding and reproductive.

**Example 2: growth curve preliminary screening**

The thirty yeast strains obtained after isolation and purification in Example 1 were inoculated into a test tube containing 5 mL of YPD liquid medium at 30°C and 180 rpm for 20 h, then inoculated into a 100-well culture plate containing 300 $\mu$L of YPD liquid medium according to an inoculation amount of 3%, and prepared for on-machine determination on a Bioscreen instrument; and parameters were set: a temperature of 30°C, a time of 24 h, and a wavelength of 600 nm; data was measured once every 30 min; a specific growth rate was analyzed; and the specific formula was as follows:

$$\text{Specific growth rate } (\mu) = \frac{LNOD2 - LNOD1}{t2 - t1}$$

OD1: a corresponding $OD_{600}$ value at t1;
OD2: a corresponding $OD_{600}$ value at t2;
t2: an end time of a logarithmic growth phase; and
t1: a start time of the logarithmic growth phase.

A screening criterion was that the specific growth rate was greater than or equal to 0.3 $h^{-1}$. Among the thirty yeast strains, six yeast strains with growth dominance were preferentially obtained, and were named as a strain 1, a strain 2, a strain 3, a strain 4, a strain 5 and a strain 6 respectively. Species information of the six yeast strains was *Saccharomyces cerevisiae* according to the results in Example 1. The specific growth rates of the six yeast strains with growth dominance were shown in Table 3 below.

Table 3 Specific growth rates of six yeast strains with growth dominance

| Strain name | Specific growth rate $\mu(h^{-1})$ |
|---|---|
| Strain 1 | 0.3085 |
| Strain 2 | 0.3217 |
| Strain 3 | 0.3173 |
| Strain 4 | 0.3200 |
| Strain 5 | 0.3049 |
| Strain 6 | 0.3108 |

**Example 3: shake flask fermentation screening**

Biomass, protein, RNA and other indexes of the six yeast strains with growth dominance in Example 2 were determined, with the existing *Saccharomyces cerevisiae* FX-2 as a control. Seven yeast strains were inoculated into test tubes each containing 5 mL of YPD liquid medium at 30°C and 180 rpm for 20 h, and then inoculated into triangular flasks each containing 300 mL of YPD liquid medium according to an inoculation amount of 0.6%, cultured at 30°C and 180 rpm for 18 h, and centrifuged; a supernatant was discarded; yeast milk was collected; and biomass, protein, RNA and other indexes were determined.

Screening criteria for physiological indexes of the yeast milk were as follows: the biomass reached 110-120% of the biomass of the control strain; the protein content reached 100-110% of the protein content of the control strain; and the RNA content reached 110-120% of the RNA content of the control strain. A relative gain was calculated according to the following formula:

$$\text{Relative gain}(\%) = \frac{\text{certain physiological index content of screened strain}}{\text{certain physiological index content of control strain}} \times 100$$

1. Biomass determination

Weighing the yeast milk was known as biomass determination.

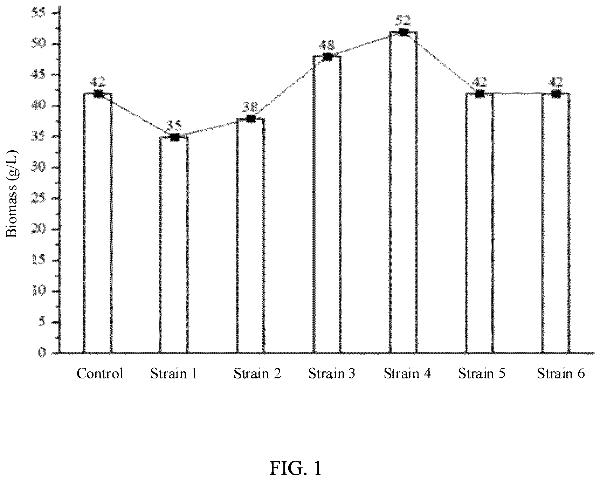

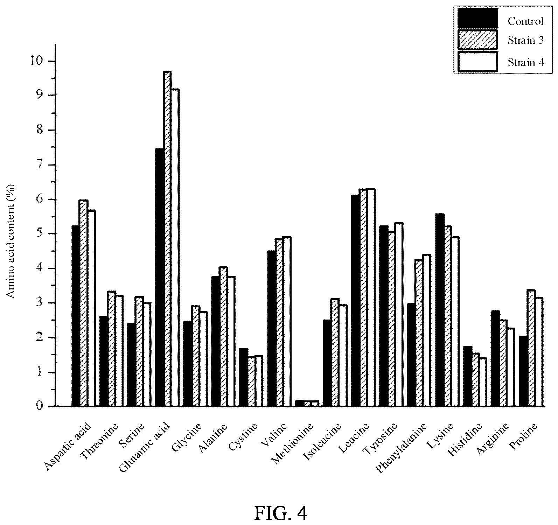

The determination results of the biomass indexes were shown in FIG. 1 and FIG. 4, wherein the biomass of the control strain was 42 g/L, the strain 3 (48 g/L) and the strain 4 (52 g/L) each have a biomass superior to this index of the control strain, which were 14.3% and 23.8% higher than that of the control strain, respectively; and the biomass levels of the strain 5 and the strain 6 were consistent with that of the control strain.

2. Dry substance detection method

A sample was dried directly in a 103±2°C drying oven; and after the loss of moisture, the resulting mass percentage was a dry substance percentage.

3. Yeast milk protein detection method

1 g of yeast milk (accurate to 0.0002 g) was weighed accurately and placed into a digestive tube, added with 2.5 g of digestive powder, slowly added with 10 mL of concentrated sulfuric acid along a wall of the tube, then placed on a digestion device, and digested for about 3 h until it is smokeless; the solution became clear and light yellow, and then continued to be heated for 10 min. The digestive tube was taken out, placed for cooling, rinsed for the tube wall with about 30 mL of distilled water, cooled again, then transferred into a 100 mL volumetric flask, rinsed with a small amount of water for three times, poured into a volumetric flask, added with water to a constant volume scale, and shaken well to obtain a digestive solution for later use. 25 mL of the digestive solution was pipetted accurately into the digestive tube and placed on a distillation device. 25 mL of boric acid solution was added to a triangular flask, added with 4-6 drops of methyl red-bromocresol green mixed indicator as a receiving solution and placed on a receiving table. A circulating water valve was opened; 25 mL of sodium hydroxide solution was added to a digestive tube; a steam switch was switched on, and the receiving table was lift, such that a receiving tube was immersed in the receiving solution and distilled till the receiving solution was 150-200 mL; the receiving table was put down, and steam and circulating water was turned off; a receiving nozzle was rinsed with distilled water; and the receiving bottle was taken out. The receiving solution was titrated with a 0.05 mol/L sulfuric acid standard solution; an end point was reached as the color became reddish from green; a blank test was done at the same time according to the above method; and the protein content was calculated according to the following formula:

$$X = \frac{C(V1 - V2) \times 0.01401}{W \times Ds \times 25 \div 100} \times 100 \times 6.25$$

in which:

X represented a percentage of protein in the sample, %;
C represented a concentration of a sulfuric acid standard solution, mol/L;
V1 represented a volume of the sulfuric acid standard solution consumed by the titration of the sample, mL;
V2 represented a volume of the sulfuric acid standard solution consumed by the titration of a control, mL;
0.01401 referred to a mass of nitrogen expressed in grams equivalent to 1.00 mL of sulfuric acid [C(1/2H₂SO₄)=1.000 mol/L] standard solution;
W represented a mass of the sample, g;
Ds represented a percentage of dry substance in the sample, %; and
6.25 represented a conversion factor of nitrogen to crude protein.

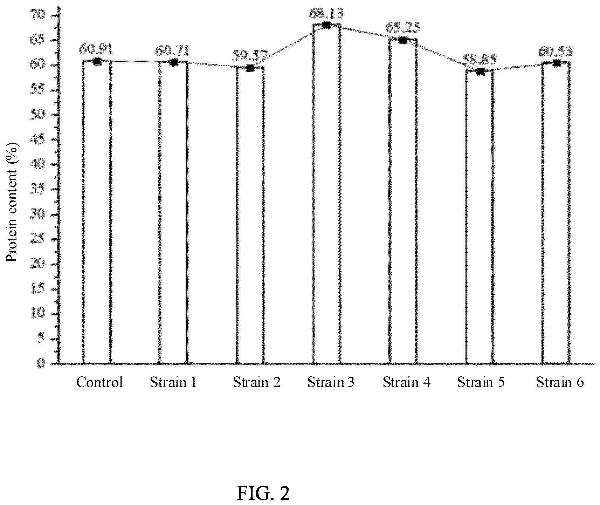

The determination results of protein contents were shown in FIG. 2 and Table 4. The protein content of the control strain was 60.91%, and the strain 1, the strain 3, the strain 4 and the strain 6 each had the protein content of greater than 60%, among which the strain 3 and the strain 4 each had the protein content exceeding the protein content of the control, the protein content of the strain 3 was 68.13%, and the protein content of the strain 4 was 65.25%.

4. RNA detection method

0.4-0.8 g of yeast milk sample was weighed, placed in a centrifuge tube and then weighed, added with 8 mL of cold 0.25 mol/L $HCLO_4$, shaken and mixed well, immediately placed into 4°C cold water, stood for 15 min, and centrifuged at 4000 rpm for 10 min; a supernatant was discarded; 5 mL of 0.5 mol/L $HCLO_4$ was added to precipitates, shaken and mixed well; the centrifuge tube was placed into a 70°C water bath, heat-preserved for 15 min, shaken once every 5 min, and centrifuged at 4000 rpm for 10 min; and 1 mL of supernatant was pipetted, added with distilled water to a constant volume of 100 mL, and mixed well. A cuvette was rinsed with a sample to be measured, and a spectrophotometer was placed after the cuvetted was filled with the sample. The surface was wiped to be clean, an absorbance value at 260 nm was measured, distilled water was used as a blank, and the absorbance was recorded; and a formula was as follows:

$$RNA(\%) = \frac{A \times 100 \times 0.03365 \times 5 \times 100}{m \times Ds}$$

in which:

A represented an absorbance of a sample solution;
m represented a mass of the weighed sample, mg;
Ds represented a dry substance of the sample;
5 represented a volume of solution after 0.5 mol/L $HCLO_4$ was added; and
0.03365 corresponded to a content of RNA in the solution to be measured when the absorbance was 1, mg/100mL.

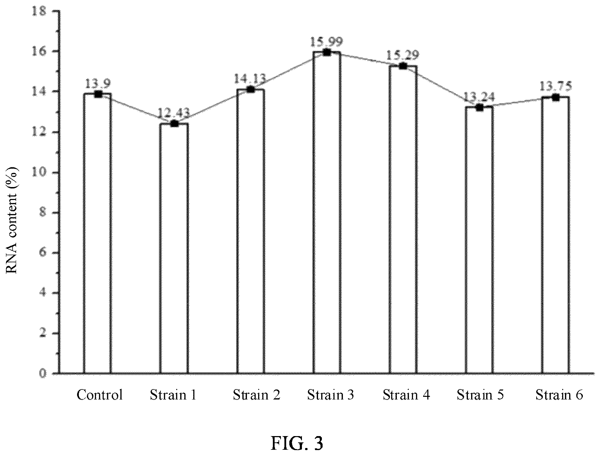

The determination results of RNA indexes were shown in FIG. 3 and Table 4, the RNA content of the control strain was 13.9%, and the strain 2, the strain 3 and the strain 4 each had the RNA content exceeding that of the control.

Table 4 Physiological indexes and relative gain (%) of yeast milk

|  | Biomass (g/L) | Relative gain (%) | Protein (%) | Relative gain (%) | RNA(%) | Relative gain (%) |
|---|---|---|---|---|---|---|
| Control | 42 | 100% | 60.91 | 100% | 13.9 | 100% |
| Strain 1 | 35 | 83% | 60.71 | 100% | 12.43 | 89% |
| Strain 2 | 38 | 90% | 59.57 | 98% | 14.13 | 102% |
| Strain 3 | 48 | 114% | 68.13 | 112% | 15.99 | 115% |
| Strain 4 | 52 | 124% | 65.25 | 107% | 15.29 | 110% |
| Strain 5 | 42 | 100% | 58.85 | 97% | 13.24 | 95% |
| Strain 6 | 42 | 100% | 60.53 | 99% | 13.75 | 99% |

As shown in Table 4 above, the biomass index of each of the strains 3 and 4 reached 110-120% of the biomass index of the preferred standard; the protein index of each of the strain 1, the strain 3 and the strain 4 reached 100-110% of the protein index of the preferred standard; and the RNA index of each of the strains 3 and 4 reached 110-120% of the RNA index of the preferred standard. In summary, the biomass, protein and RNA content indexes of the strains 3 and 4 were superior to those of the control strain, and subsequent indexes could be determined.

**Example 4: glutamic acid content analysis**

The differences in amino acid components of yeast strains led to abundant flavor characteristics of metabolites of the strains, among which glutamic acid was representative amino acid of umami.

The strain 3, the strain 4 and the control strain were each inoculated into a test tube containing 5 mL of YPD liquid medium, cultured at 30°C and 180 rpm for 20 h, then each inoculated into a triangular flask containing 300 mL of YPD liquid medium according to an inoculation amount of 0.6%, cultured at 30°C and 180 rpm for 18 h, and centrifuged; a supernatant was discarded, and yeast milk was collected; 0.5-1 g of yeast milk was weighed, placed in a 50 mL volumetric flask, added with 20 mL of sulfosalicylic acid, treated ultrasonically until fully dissolved, metered to a constant volume scale of a 50 mL, mixed well, and stood for 1 h; 1 mL of supernatant was then pipetted accurately and placed into a 25 mL volumetric flask, added with sodium citrate buffer to a constant volume scale, mixed well and filtered to an injection bottle through a 0.45 $\mu$m microporous filter membrane; a mixed amino acid standard solution was selected as an external standard; and the contents of various amino acid components were detected by an automatic amino acid analyzer according to the following formula:

the content of amino acids in a sample determination solution:

$$Ci = \frac{Cs}{As} \times Ai$$

in which: Ci represented the content of the amino acid i in the sample determination solution, nmol/L;

Ai represented an area of the amino acid i in the sample determination solution, mg;
As represented a peak area of the amino acid s in an amino acid standard working solution;
Cs represented the content of the amino acid s in the amino acid standard working solution, nmol/L;

the content of amino acids in a sample:

$$Xi = \frac{Ci \times F \times V \times M}{m \times 10^9} \times 100$$

in which: Xi represented the content of the amino acid i in the sample, g/100g;

Ci represented the content of the amino acid i in the sample determination solution, nmol/L;
F represented a dilution factor;
V represented a constant volume of the sample, mL;
M represented a molar mass of the amino acid i, g/mol;
m represented a weighing amount, g;
$10^9$ represented a coefficient of converting the sample content from ng to g; and
100 represented a conversion factor.
Cs represented the content of the amino acid s in the amino acid standard working solution, nmol/L.

FIG. 4 showed the contents of amino acid components in the strain 3, the strain 4 and the control strain, and the results showed that glutamic acid accounted for the highest proportion of seventeen free amino acids. Table 5 showed the content of glutamic acid components in the strain 3, the strain 4 and the control strain, in which the glutamic acid content of the strain 3 was 130% of the glutamic acid content of the control, and the glutamic acid content of the strain 4 was 123% of the glutamic acid content of the control, that is, the glutamic acid content of the strain 3 reached a preferred standard of 125-130%.

Table 5 Content of glutamic acid components in yeast strain (%)

|  | Glutamic acid (%) | Relative gain |
|---|---|---|
| Control | 7.45 |  |
| Strain 3 | 9.69 | 130% |
| Strain 4 | 9.18 | 123% |

**Example 5: succinic acid content analysis**

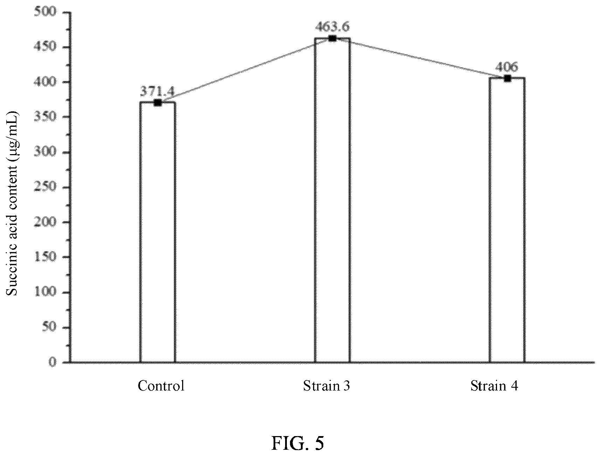

The strain 3, the strain 4 and the control strain were activated and then each cultured in a 250 mL flask, with YPD liquid as a medium, cultured in a 20 L fermentation tank, then washed, and subjected to suction filtration, and pressing filtration; microbial cells were isolated and collected to obtain yeast milk; and deionized water was added according to a mass volume ratio of the yeast milk to the deionized water of 1:10, adjusted for pH to 6.5, and homogenized twice under a high pressure. The yeast was stirred at 45°C, autolyzed for 36 h, and centrifuged at 9000 rpm; a supernatant was taken, filtered through a 0.22 μm syringe filter, and injected into a liquid chromatograph for determination. At the same time, a succinic acid standard product was selected for liquid chromatography. FIG. 5 and Table 6 showed the succinic acid contents of the strain 3, the strain 4 and the control strain.

Example 6: succinic acid content

|  | Succinic acid (μg/mL) | Relative gain |
| --- | --- | --- |
| Control | 371.4 |  |
| Strain 3 | 463.6 | 125% |
| Strain 4 | 406 | 109% |

The results of FIG. 5 and Table 6 showed that the succinic acid content of the strain 3 was 463.6 μg/mL and the relative gain was 125%, reaching a preferred standard of 110-120%.

**Example 6: strain identification**

A genome of the strain 3 was extracted, a 26S rDNA sequence of the yeast was amplified by taking NL1 (5'-GCATATCAATAAGCGGAGAGGAA AA G-3') and NL4 (5'-GGTCCGTGTTTCAAGACGG -3') as primers, and a 26S rDNA gene sequence of the strain 3 was obtained after 1% gel electrophoresis detection and sequencing, as shown in SEQ ID NO. 1. A gene sequence for SEQ ID NO.1 was as follows:

> acggggatgcttagtaacggcgagtgaagcggcaaaagctcaaatttgaaatctggtaccttcggtgcccgagttgtaatttggagagggcaa
> ctttggggccgttccttgtctatgttccttggaacaggacgtcatagagggtgagaatcccgtgtggcgaggagtgcggttctttgtaaagtgcc
> ttcgaagagtcgagttgtttgggaatgcagctctaagtgggtggtaaattccatctaaagctaaatattggcgagagaccgatagcgaacaagt
> acagtgatggaaagatgaaaagaactttgaaaagagagtgaaaaagtacgtgaaattgttgaaagggaagggcatttgatcagacatggtgtt
> ttgtgccctctgctccttgtgggtaggggaatctcgcatttcactgggccagcatcagttttggtggcaggataaatccataggaatgtagcttgc
> ctcggtaagtattatagcctgtgggaatactgccagctgggactgaggactgcgacgtaagtcaaggatgctggcataatggttatatgccgcc
> cgtcttgaaccccccggacca

The strain 3 was named a *Saccharomyces cerevisiae* AMCC 30743 strain (Saccharomyces cerevisiae AMCC 30743), and the *Saccharomyces cerevisiae* AMCC 30743 strain (Saccharomyces cerevisiae AMCC 30743) was preserved. The *Saccharomyces cerevisiae* AMCC 30743 strain (Saccharomyces cerevisiae AMCC 30743) was preserved in the China Center for Type Culture Collection (CCTCC) on July 27, 2021, and had a preservation number of CCTCC NO: M 2021941.

**Claims**

1. A high-protein *Saccharomyces cerevisiae* strain and an application thereof, **characterized in that** the *Saccharomyces cerevisiae* strain is:
   a *Saccharomyces cerevisiae* AMCC 30743 strain (*Saccharomyces cerevisiae* AMCC 30743), the *Saccharomyces cerevisiae* AMCC 30743 strain (Saccharcmyces cerevisiae AMCC 30743) being preserved in the China Center for Type Culture Collection (CCTCC) on July 27, 2021, and having a preservation number of CCTCC NO: M 2021941.

2. A fermentation preparation method for the *Saccharomyces cerevisiae* strain according to claim 1, **characterized in that** the fermentation preparation method comprises the following step: culturing the *Saccharomyces cerevisiae* strain according to claim 1.

3. The fermentation preparation method according to claim 2, **characterized in that** the fermentation preparation method comprising the following steps:

(1) performing amplified culture on the *Saccharomyces cerevisiae* strain according to claim 1; and
(2) adding the product obtained in step (1) to a liquid culture medium and performing fermented culture at 26-32°C.

4. A microbial agent, **characterized in that** the microbial agent being obtained from the *Saccharomyces cerevisiae* strain according to claim 1.

5. An application of the *Saccharomyces cerevisiae* strain according to claim 1 or the microbial agent according to claim 4 in a yeast extract.

6. A yeast extract, **characterized in that** the yeast extract being prepared from the *Saccharomyces cerevisiae* strain according to claim 1 or the microbial agent according to claim 4.

7. A microbial agent, **characterized in that** the microbial agent being obtained by the fermentation preparation method according to claim 2 or 3.

8. The microbial agent according to claim 7, **characterized in that**

the microbial agent obtained by culturing in a shake flask for 15-20 h has a biomass of 48-52 g/L; or
preferably, the microbial agent has an intracellular protein content of greater than 65wt% of a dry weight of yeast cells; or
preferably, the microbial agent has an RNA content of greater than 15wt% of the dry weight of the yeast cells; or
preferably, the microbial agent has an intracellular glutamic acid content of greater than 9%; or
preferably, an intracellular succinic acid content of the microbial agent is a measured succinic acid content in a supernatant which were obtained after deionized water is added according to a mass volume ratio of the yeast milk to the deionized water of 1:10 and cells are broken, and the succinic acid content in a supernatant is greater than 460 $\mu$g/mL.

9. An application of the *Saccharomyces cerevisiae* strain according to claim 1 or the microbial agent according to claim 4 or the yeast extract according to claim 6 or the microbial agent according to claim 7 or 8 in feeds, food and health care products.

10. An application of the *Saccharomyces cerevisiae* strain according to claim 1 or the microbial agent according to claim 4 or the yeast extract according to claim 6 or the microbial agent according to claim 7 or 8 in condiments.

FIG. 1

FIG. 2

FIG. 3

FIG. 4

FIG. 5

| | INTERNATIONAL SEARCH REPORT | International application No. |
|---|---|---|
| | | **PCT/CN2022/135105** |

**A.    CLASSIFICATION OF SUBJECT MATTER**

C12N 1/16(2006.01)i;A23L 33/14(2016.01)i;A23L 33/145(2016.01)i;A23K 10/16(2016.01)i;A23K 10/18(2016.01)i;A23L 27/10(2016.01)i;C12R 1/645(2006.01)i

According to International Patent Classification (IPC) or to both national classification and IPC

**B.    FIELDS SEARCHED**

Minimum documentation searched (classification system followed by classification symbols)

IPC: C12N, A23L, A23K, C12R

Documentation searched other than minimum documentation to the extent that such documents are included in the fields searched

Electronic data base consulted during the international search (name of data base and, where practicable, search terms used)

CNTXT, WPABS, WPABSC, OETXT, USTXTC, VEN, DWPI, CJFD; CNKI, 万方数据 WANFANG DATA, PubMed, Elsevier Science, ISI WEB of Science; GenBank+EMBL; 中国专利序列数据库 Chinese Patent Sequence Database: 酿酒酵母, 高蛋白, 蛋白高, 高产蛋白, 蛋白含量高, RNA, 谷氨酸, Saccharomyces, cerevisiae, yeast, high protein, high RNA, Glutamic acid, AMCC 30743, CCTCC 2021941, 对SEQ ID NO: 1的检索, search for SEQ ID NO: 1

**C.    DOCUMENTS CONSIDERED TO BE RELEVANT**

| Category* | Citation of document, with indication, where appropriate, of the relevant passages | Relevant to claim No. |
|---|---|---|
| PX | CN 113862164 A (CHINA NATIONAL RESEARCH INSTITUTE OF FOOD & FERMENTATION INDUSTRIES CO., LTD.; ANGEL YEAST CO., LTD.) 31 December 2021 (2021-12-31)<br>    see entire document | 1-10 |
| A | CN 109198167 A (ANGEL YEAST CO., LTD.) 15 January 2019 (2019-01-15)<br>    see abstract, and claims 1-14 | 1-10 |
| A | CN 105919137 A (ANQI YEAST (CHONGZUO) CO., LTD.) 07 September 2016 (2016-09-07)<br>    see abstract, and claims 1-3 | 1-10 |
| A | CN 108220175 A (ANGEL YEAST CO., LTD.) 29 June 2018 (2018-06-29)<br>    see abstract, claims 1 and 13, and description, embodiment 3 | 1-10 |
| A | US 2020109428 A1 (THANH, L. Q.) 09 April 2020 (2020-04-09)<br>    see abstract | 1-10 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 2023年2月6日 | **17 February 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

## INTERNATIONAL SEARCH REPORT

| International application No. |
|---|
| **PCT/CN2022/135105** |

**C.    DOCUMENTS CONSIDERED TO BE RELEVANT**

| Category* | Citation of document, with indication, where appropriate, of the relevant passages | Relevant to claim No. |
|---|---|---|
| A | 陈文明 等 (CHEN, Wenmining). "YE专用高蛋白高RNA酵母菌株的筛选及鉴定 (Screening and Identification of the High-protein and High-RNA Yeast Strain for YE Product)" 食品科技 *(Food Science and Technology)*, Vol. 40, No. 4, 30 April 2015 (2015-04-30), see abstract, table 1 and table 2 | 1-10 |

Form PCT/ISA/210 (second sheet) (July 2022)

**INTERNATIONAL SEARCH REPORT**

International application No.

**PCT/CN2022/135105**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/CN2022/135105**

| Patent document cited in search report | | | Publication date (day/month/year) | Patent family member(s) | Publication date (day/month/year) |
|---|---|---|---|---|---|
| CN | 113862164 | A | 31 December 2021 | None | |
| CN | 109198167 | A | 15 January 2019 | None | |
| CN | 105919137 | A | 07 September 2016 | None | |
| CN | 108220175 | A | 29 June 2018 | None | |
| US | 2020109428 | A1 | 09 April 2020 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 106399135

- CN 108220175 A